# EUROPÄISCHE PATENTSCHRIFT

(11) **EP 1 144 016 B1**
(45) Veröffentlichungstag und Bekanntmachung des Hinweises auf die Patenterteilung: **10.12.2003**
(21) Anmeldenummer: 00974471.5
(22) Anmeldetag: 27.10.2000
(51) Int. Cl.: A61L 2/20, A61L 2/22, B65B 55/10

(54) **VERFAHREN UND VORRICHTUNG ZUM STERILISIEREN VON VERPACKUNGSBEHÄLTERN**
METHOD AND DEVICE FOR STERILISATION OF PACKAGING CONTAINERS
PROCEDE ET DISPOSITIF DE STERILISATION DE RECIPIENTS D'EMBALLAGE

(30) Priorität: 22.11.1999 DE 19956186
(43) Veröffentlichungstag der Anmeldung: 17.10.2001
(73) Patentinhaber: KRONES AG, 93068 Neutraubling (DE)
(72) Erfinder: KNIELING, Erwin, 93102 Pfatter (DE); HIENDL, Hans, 93051 Regensburg (DE)
(86) Internationale Anmeldenummer: EP0010611
(87) Internationale Veröffentlichungsnummer: WO01037886

(56) Entgegenhaltungen:
- EP-A- 0 381 841
- EP-A- 0 758 624
- DE-A- 19 642 987
- FR-A- 2 774 912
- US-A- 3 481 109

## Beschreibung

Die Erfindung betrifft ein Verfahren zum Sterilisieren von Verpackungsbehältern gemäß dem Oberbegriff des Anspruchs 1 sowie eine hierfür geeignete Vorrichtung gemäß dem Oberbegriff des Anspruchs 13.

Es ist bereits ein derartiges Verfahren bekannt, bei dem ein H2O2-Dampfgemisch über eine Zweistoffdüse und eine Heizkammer sowie eine Rohrleitung in eine Sterilisationskammer eingeleitet wird (DE 39 00 448 A1). Die zu behandelnden Verpackungsbehälter in Form von konischen Bechern aus Kunststoff werden durch die Sterilisationskammer hindurchbewegt und dabei von dem erwärmten H2O2-Dampfgemisch diffus beaufschlagt. Zusätzlich wird in die Sterilisationskammer heiße Luft eingeblasen die das H2O2 chemisch zersetzen soll. Anschließend werden die Reste des Sterilisationsmittels bzw. des Gemisches aus der Sterilisationskammer abgesaugt.

Dieses bekannte Verfahren hat einen sehr komplexen Ablauf und ist entsprechend aufwendig in der Durchführung. Aufgrund des ungerichteten, diffusen Einleitens des H2O2-Dampfgemisches in die Sterilisationskammer ist es nur für Verpackungsbehälter mit relativ großer Mündungsöffnung geeignet, nicht dagegen für Verpackungsbehälter mit kleiner Mündung wie z.B. Getränkeflaschen aus Kunststoff, wie sie in der Getränkeindustrie zunehmend Verwendung finden.

Auch ist bereits ein speziell zum Sterilisieren von PET-Flaschen eingerichtetes Verfahren bekannt, bei dem in ein Druckluft führendes Rohr mittels einer Vernebelungsdüse kontinuierlich Peressigsäure mit einer Konzentration von 0,1 bis 1,5 Prozent eingesprüht wird (DE 198 08 318 A1). Das so gebildete Aerosol wird anschließend in einem Wärmetauscher erhitzt und schließlich über Rohrleitungen und Ein-Komponenten-Düsen in die auf dem Kopf stehenden PET-Flaschen eingeleitet. Nach der Einwirkung wird das Sterilisationsmittel durch Ausspritzen der PET-Flaschen mittels demineralisiertem Wasser entfernt.

Mit diesem bekannten Verfahren können zwar auch enghalsige Verpackungsbehälter sterilisiert werden, es ist jedoch nur mit hohem apparativen Aufwand realisierbar. Problematisch ist auch das Verhalten bei den unvermeidlichen Betriebsunterbrechungen, während denen das erwärmte Aerosol in den langen Rohrleitungen zu den Düsen kondensieren kann. Dies führt zu nicht einwandfrei sterilisierten Verpackungsbehältern nach der Unterbrechung.

Der Erfindung liegt die Aufgabe zugrunde, ein einfach zu realisierendes und betriebssicher arbeitendes Verfahren zum Sterilisieren von Verpackungsbehältern zu schaffen, mit dem auch Verpackungsbehälter mit relativ kleiner Mündungsöffnung zuverlässig sterilisiert werden können. Außerdem soll eine kostengünstig aufgebaute Vorrichtung zur Durchführung des Verfahrens aufgezeigt werden.

Diese Aufgabe wird hinsichtlich des Verfahrens durch die Merkmale des Anspruchs 1 und hinsichtlich der Vorrichtung durch die Merkmale des Anspruchs 13 gelöst.

Beim erfindungsgemäßen Verfahren erfolgt sowohl die Vernebelung bzw. Verdampfung als auch die Erwärmung des flüssigen Desinfektionsmittels allein durch die Mischung mittels Wasserdampf. Dabei kann die Erwärmung in einfachster Weise durch Auswahl der Temperatur und Menge des Wasserdampfes definiert werden. Die dabei erfolgende "Verdünnung" des Desinfektionsmittels kann gleichfalls auf einfache Weise durch entsprechende Erhöhung der Ausgangskonzentration des der Mischdüse zugeführten Desinfektionsmittels kompensiert werden, so dass das an den vorzugsweise Raumtemperatur aufweisenden Verpackungsbehältern kondensierte Gemisch die gewünschte Konzentration aufweist.

Aufgrund des direkten Aufsprühens des Desinfektionsmittel-Wasserdampf-Gemisches auf die Verpackungsbehälter durch die Mischdüsen sind keine Heizkammern, Rohrleitungen usw. erforderlich, in denen das Gemisch kondensieren könnte. Es ist daher ein taktweiser Betrieb der Mischdüsen möglich und auch längere Betriebsunterbrechungen haben keine negativen Auswirkungen. Durch entsprechende Gestaltung der Mischdüsen und der sich daraus ergebenden Strahlform des nebelartigen Gemisches kann dieses sowohl in enge Mündungsöffnungen als auch breitflächig auf die Außenwand von Verpackungsbehältern aufgeblasen bzw. aufgenebelt werden. Außerdem ist eine exakte Dosierung des auf einen Verpackungsbehälter aufgedüsten Gemisches und daher ein besonders sparsamer Verbrauch möglich.

Vorteilhafte Weiterbildungen der Erfindung sind in den Unteransprüchen enthalten.

Im nachstehenden wird ein Ausführungsbeispiel der Erfindung anhand der Zeichnungen beschrieben. Es zeigen:
- Fig.1: die schematische Draufsicht auf eine Vorrichtung zum Sterilisieren von Verpackungsbehältern
- Fig. 2: den Schnitt AB nach Fig. 1 in vergrößerter Darstellung
- Fig. 3: die Einzelheit X in vergrößerter Darstellung
- Fig. 4: den Schnitt CD nach Fig.1 in vergrößerter Darstellung
- Fig. 5: den Schnitt EF nach Fig. 1 in vergrößerter Darstellung.

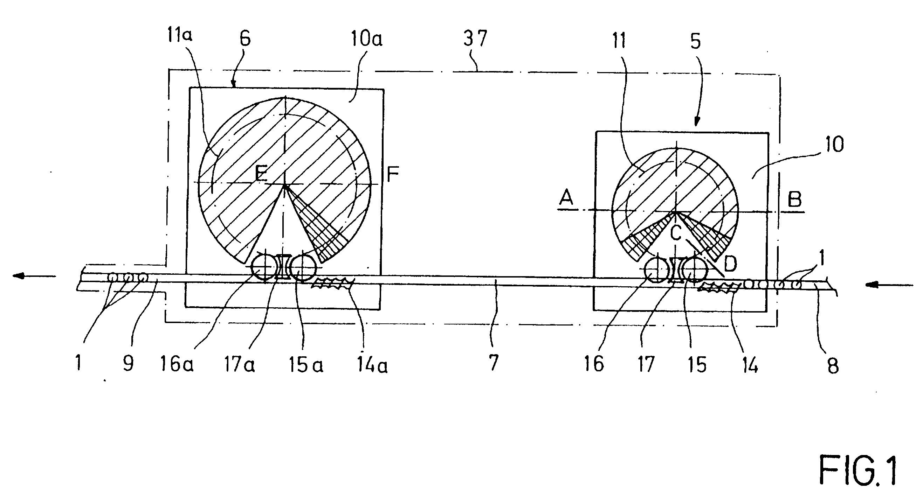

Die Vorrichtung nach Fig. 1 bis 5 ist zum sterilisieren von Verpackungsbehältern in Form von PET- Flaschen für Getränke, im nachstehenden kurz Flaschen 1 genannt, eingerichtet. Sie umfaßt im Wesentlichen eine Injektionsmaschine 5, eine Spülmaschine (Rinser) 6 und ein die beiden Maschinen verbindendes Förderband 7. Die zu sterilisierenden Flaschen 1 werden der Vorrichtung durch ein Zuförderband 8 aufrecht stehend zugeführt; die sterilisierten Flaschen 1 werden von der Vorrichtung durch ein Abförderband 9 aufrecht stehend abgeführt.

Die Injektionsmaschine 5 besitzt ein stationäres Gehäuse 10, auf dem ein Drehtisch 11 mit senkrechter Drehachse 12 gelagert ist. Am Umfang des Drehtisches 11 ist gleichmäßig verteilt eine Anzahl von elastischen Greifzangen 13 gemäß dem europäischen Patent 721 808 starr befestigt. Diese Greifzangen 13 halten die Flaschen 1 unterhalb des Halskragens in aufrechter Position und mit der Mündung nach oben weisend fest, während sie mit dem Drehtisch 11 auf einer Kreisbogenbahn umlaufen. Die Zu- und Abfuhr der aufrecht stehenden Flaschen 1 zu den Greifzangen 13 erfolgt durch eine Einteilschnecke 14, einen Einlaufstern 15, einen Auslaufstern 16 und einen Führungsbogen 17, wie dies im genannten europäischen Patent 721 808 detailliert beschrieben ist. Ein Verschwenken der Flaschen um 180° findet jedoch nicht statt. Diese behalten im schraffiert angedeuteten Behandlungsbereich durchgehend ihre aufrechte Normalposition.

Auf dem Drehtisch 11 der Injektionsmaschine 5 ist weiter eine Anzahl von Zwei-Komponenten-Zerstäubungsdüsen mit einem Sprühwinkel von ca. 20°, im nachstehenden kurz Mischdüsen 2 genannt, starr befestigt. Genauer gesagt ist über jeder Greifzange 13 konzentrisch zu einer von dieser gehaltenen Flasche 1 jeweils eine Mischdüse 2 senkrecht nach unten gerichtet und mit geringem Abstand (ca. 2 cm) zur Mündungsöffnung der Flasche 1 angeordnet.

Der erste Kanal 18 jeder Mischdüse 2 ist über erste Leitungen 22 unter Zwischenschaltung eines ersten Steuerventils 20 und eines konzentrisch zur Drehachse 12 angeordneten Drehverteilers 27 mit einem stationären Wasserdampferzeuger 24 verbunden. Dieser liefert Wasserdampf mit einem Überdruck von 2 Bar und einer Temperatur von 121° Celsius. Der zweite Kanal 19 jeder Mischdüse 2 ist über zweite Leitungen 23 und unter Zwischenschaltung eines zweiten Steuerventils 21 sowie des Drehverteilers 27 und einer Pumpe 25 mit einem stationären Vorratstank 26 für unter Raumtemperatur stehendes Desinfektionsmittel 3 verbunden. Dieses besteht aus einer wässrigen Lösung mit 4% eines Desinfektionsmittelkonzentrats sowie 0,04% Tensid zur Verbesserung der Benetzung. Dieses flüssige Desinfektionsmittel 3 wird durch die Pumpe 25 mit einem Überdruck von 2 Bar den Mischdüsen 2 zugeführt. Das Desinfektionsmittel enthält als keimtötende Bestandteile 4000 ppm H202 und 2500 ppm Peressigsäure.

Mit den vorbeschriebenen Einrichtungen der Injektionsmaschine 5 wird ins Innere der Flaschen 1 ein vernebeltes Gemisch aus flüssigem Desinfektionsmittel 3 und Wasserdampf 4 eingeblasen. Um auch die Außenfläche der Flaschen 1 zu sterilisieren, sind im Anschluß an den Einlaufstern 15 mehrere Mischdüsen 2a stationär und mit geringem Abstand seitlich an der Bewegungsbahn der Flaschen 1 angeordnet. Diese Mischdüsen 2a sind -abgesehen vom hier nicht erforderlichen Drehverteiler 27- in gleicher Weise wie die mit dem Drehtisch umlaufenden Mischdüsen 2 über Leitungen 23 und Steuerventile 21 mit dem Vorratstank 26 verbunden und sind horizontal ausgerichtet. Außerdem sind sie über Leitungen 38 mit einer Quelle für sterile Druckluft verbunden.

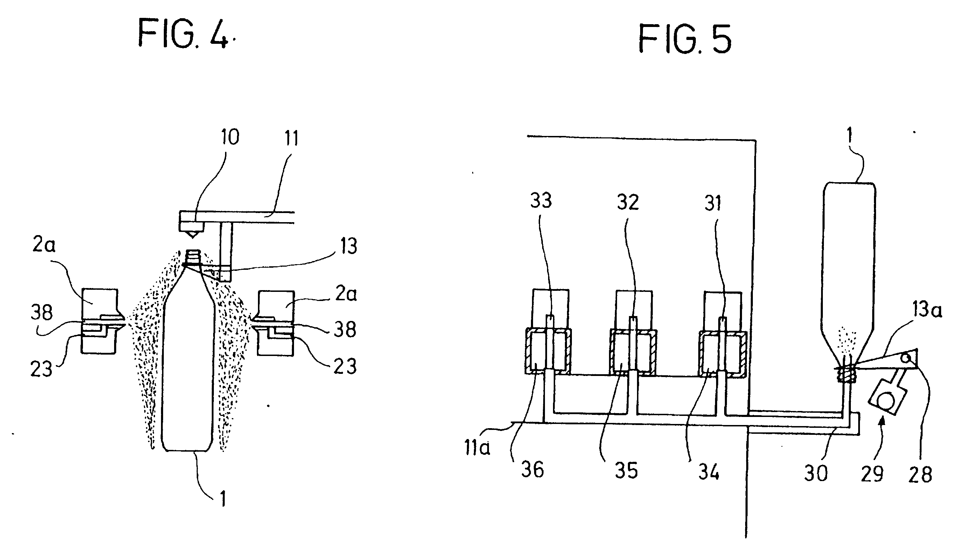

Die Spülmaschine 6 nach Fig. 1 und 5 weist einen teilweise ähnlichen Aufbau auf wie die Injektionsmaschine 5 nach Fig. 1 bis 4. Übereinstimmende Teile sind daher mit der gleichen Bezugsziffer und dem Zusatz "a" versehen. Die elastischen Greifzangen 13a sind hier um horizontale Schwenkachsen 28 mittels einer Kurvensteuerung 29 schwenkbar, wie dies im Detail in der europäischen Patentschrift Nr. 721 808 beschrieben ist. Die Flaschen 1 stehen daher während des größten Teils ihrer Umlaufbewegung mit dem Drehtisch 11 auf dem Kopf bzw. weisen mit ihrer Mündungsöffnung nach unten. Im Bereich des Einlaufsterns 15a und des Auslaufsterns 16a dagegen weisen sie ihre aufrechte Normalposition auf.

Die Behandlung der Flaschen 1 in der Spülmaschine 6 erfolgt durch auf dem Drehtisch 11a angeordnete, den Greifzangen 13a zugeordnete Düsen 30, die in die Flaschen 1 etwas einführbar sind. Jede Düse 30 ist über jeweils ein Steuerventil 31, 32, 33 an insgesamt drei Kanäle 34, 35, 36 angeschlossen, die verschiedene Spülmedien enthalten. Im vorliegenden Falle sind dies Sterilluft, Sterilwasser und flüssiges Desinfektionsmittel 3, vorzugsweise das gleiche wie im Vorratstank 26. Die Düse 30 ist als Ein- oder Mehrrohrdüse ausgebildet, so dass ggf. auch gleichzeitig verschiedene Spülmedien in die Flasche 1 eingeleitet werden können.

Das Förderband 7 verbindet den Auslaufstern 16 der Injektionsmaschine 5 mit der Einlaufschnecke 14a und dem Einlaufstern 15a der Spülmaschine 6. Seine Länge ist derart bemessen, dass sich bei Nennleistung der Vorrichtung die gewünschte Einwirkungszeit für den in der Injektionsmaschine 5 in die Flasche eingedüsten Desinfektionsmittel-Wasserdampf-Nebel ergibt. Im Normalbetrieb sind die Injektionsmaschine 5, das Förderband 7 und die Spülmaschine 6 durch nicht gezeigte Antriebsorgane synchron zueinander angetrieben, so dass sich ein störungsfreier, kontinuierlicher Transport der Flaschen 1 vom Zuförderer 8 bis zum Abförderer 9 ergibt. Die gesamte Vorrichtung ist in einer strichpunktiert angedeuteten Kammer 37 angeordnet, die mit Sterilluft der Klasse 100 beaufschlagt ist. Eine Reinfektion der sterilisierten Flaschen 1 wird so verhindert.

Mit der vorbeschriebenen Vorrichtung wird das nachfolgend beschriebene Sterilisationsverfahren durchgeführt:

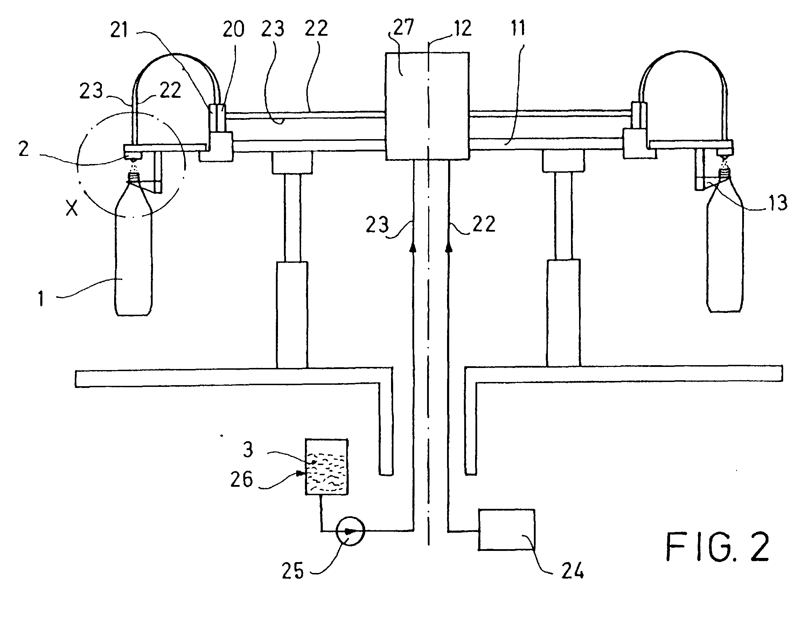

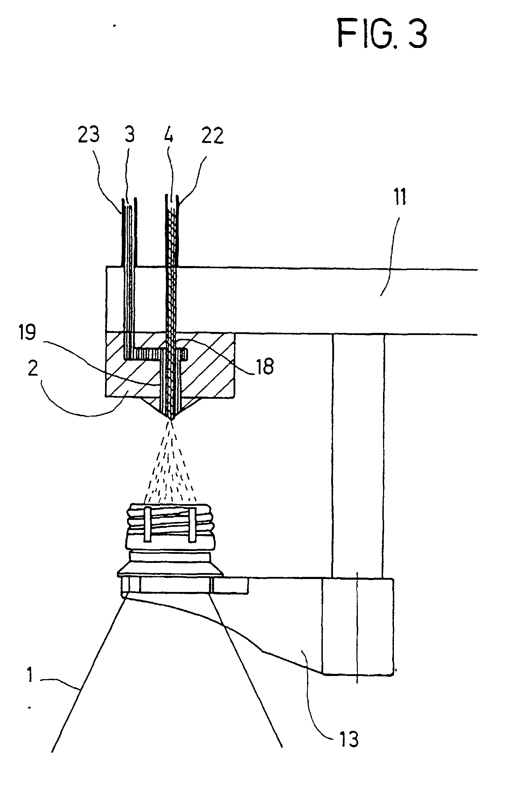

Eine von einer nicht gezeigten Streckblasmaschine aufrechtstehend angelieferte neue Flasche 1 wird durch den Zuförderer 8 zugeführt, durch die Einlaufschnecke 14 eingetaktet und vom Einlaufstern 15 an einen Greifer 13 der Injektionsmaschine 5 übergeben. Sie befindet sich nun mittig in der in Fig. 2 und 3 gezeigten Position unter der der Greifzange 13 zugeordneten Mischdüse 2, die von der Flaschenmündung einen Abstand von zwei bis drei Zentimetern aufweist. Nunmehr werden die beiden Steuerventile 20 und 21 der Mischdüse 2 für einen Zeitraum von 1,5 Sekunden synchron geöffnet. Dabei wird der Mischdüse 2 gleichzeitig aus dem Vorratstank 26 flüssiges Desinfektionsmittel 3 mit der bereits beschriebenen Zusammensetzung bei Raumtemperatur und einem Überdruck von zwei Bar sowie vom Dampferzeuger 24 Wasserdampf mit einem Überdruck von zwei Bar und einer Temperatur von 121° getrennt zugeführt. Beim Austritt aus den Kanälen 18, 19 der Mischdüse 2 werden die beiden Komponenten intensiv vermischt wobei sich ein energiereicher Gemischstrahl mit einer Temperatur von ca. 60 bis 80°C und einem Öffnungswinkel von rund 20° einstellt. Der Gemischstrahl enthält im wesentlichen fein vernebeltes Desinfektionsmittel, Wasserdampf und ggf. verdampftes Desinfektionsmittel und Wassertröpfchen. Durch die katalytisch wirkende Temperaturerhöhung bzw. Wärmezufuhr wird das Desinfektionsmittel 3 optimal aktiviert.

Die Mischdüse 2 ist derart angeordnet und ausgebildet, dass der durch sie erzeugte Gemischstrahl im wesentlichen senkrecht nach unten durch die Mündungsöffnung ins Innere der Flasche 1 eindringt und diese vollständig ausfüllt. Da die Flasche 1 lediglich Raumtemperatur aufweist, schlägt sich der größte Teil des Gemisches sofort in Form feinster Tröpfchen auf der inneren Flaschenwandung nieder und bildet einen geschlossenen, hochwirksam keimtötenden Kondensatfilm. Die Innentemperatur der Flasche 1 steigt hierbei aufgrund des insgesamt nur geringen Wärmeinhalts des eingesprühten Gemisches auf ca. 45°C an.

Durch den vorbeschriebenen Vorgang wird außerdem die ursprünglich in der Flasche 1 enthaltene Umgebungsluft größtenteils aus dem Flascheninnenraum verdrängt. Am Ende der 1,5 Sekunden Sprühzeit werden die beiden Steuerventile 20, 21 synchron geschlossen. Die beschriebenen 1,5 Sekunden Sprühzeit beanspruchen den in Fig. 1 kreuzweise schraffierten Umlaufbereich des Drehtisches 11. Nunmehr schließt sich eine Wirkzeit von 5 Sekunden an, die durch den einfach schraffierten Bereich des Drehtisches 11 markiert ist. Hierbei wirkt das auf die Flascheninnenwand aufgenebelte Desinfektionsmittel 3 intensiv keimtötend. Auch der äußere Bereich der Flaschenmündung wird durch austretendes Gemisch mit behandelt.

In der Endphase des Umlaufbereichs der Flasche 1 mit dem Drehtisch 11 erfolgt nochmals in gleicher Weise ein 1,5 Sekunden dauerndes Einsprühen eines Desinfektionsmittel-Wasserdampf-Gemisches durch die gleiche Mischdüse 2. Dabei erwärmt sich die Flasche innen auf ca. 58°C. Die kritische Temperatur für PET-Flaschen von ca. 65°C wird somit nicht annähernd erreicht. Danach wird die Flasche 1 vom Auslaufstern 16 und vom Führungsbogen 17 erfasst und auf dem Förderband 7 abgestellt. Es folgt eine von der Länge des Förderbandes 7 abhängige Einwirkungszeit, die je nach Art der Flasche, des gewünschten Sterilisationseffekts und des verwendeten Desinfektionsmittels variiert werden kann und vorzugsweise im Bereich von 5 bis 10 Sekunden liegt.

Aufgrund der Direktbeaufschlagung des Flascheninneren durch die Mischdüse 2 und deren exakter Zeitsteuerung ist eine exakte Dosierung des eingedüsten Desinfektionsmittels 3 und des Wasserdampfs 4 möglich. So wird beispielsweise beim Sterilisieren einer 1,5 Liter PET-Flasche mit den geschilderten Parametern und einer entsprechend dimensionierten Mischdüse 2 während der 1,5 Sekunden dauernden Einsprühphase in die Flasche 1 jeweils 3,4 Milliliter Desinfektionsmittel 3 und 1,4 Gramm Wasserdampf 4 eingedüst. Bei einer Leistung von 19.000 Fl/h ergibt sich dann ein stündlicher Verbrauch von 129,2 Litern Desinfektionsmittel 3 und 53,2 Kilogramm Dampf 4. Die erzielbare Keimabtötungsrate liegt bei 99,99% bis 99,999%, je nach Art der verwendeten Testkeime. Dies genügt allen Anforderungen beim sterilen Abfüllen von Getränken in PET-Flaschen.

Hierzu kommt ggf. noch ein Verbrauch von rund 33 Litern pro Stunde an Desinfektionsmittel 3 für die externe Sterilisation der Flaschen 1 durch die Düsen 2a im Einlaufbereich des Drehtisches 11, durch die erforderlichenfalls eine Reinfektion des Flascheninneren durch an der Flaschenaußenseite sitzende Keime zuverlässig verhindert wird.

Am Ende des Förderbandes 7 wird die innen und außen einen Desinfektionsmittel-Film tragende Flasche 1 durch die Einlaufschnecke 14a eingetaktet und vom Einlaufstern 15a in aufrechter Normalposition an eine Greifzange 13a der Spülmaschine 6 übergeben. Daraufhin wird die Greifzange 13a während der Rotation des Drehtisches 11a durch die Steuereinrichtung 29 um 180° verschwenkt, so dass die Flasche 1 schließlich mit ihrer Mündung senkrecht nach unten weist. Dabei dringt die Düse 30 einige Millimeter in die Flaschenmündung ein (Fig. 5). Nunmehr wird durch eine entsprechende Ansteuerung der Steuerventile 31, 32, 33 als erstes kurzzeitig flüssiges Desinfektionsmittel in die Flasche 1 eingespritzt (kreuzweise schraffierter Bereich) und dann die Flasche 1 abwechselnd mit sterilem Wasser und steriler Luft gespült (einfach schraffierter Bereich) bis auch die letzten Reste des Desinfektionsmittels praktisch vollständig aus der Flasche 1 entfernt worden sind. Danach wird die Flasche 1 zurück in ihre Normalposition verschwenkt, vom Auslaufstern 16a übernommen und auf dem Abförderer 9 in aufrechter Normalposition abgestellt. Von diesem wird sie zu einer nicht gezeigten Steril-Abfüll- und Verschließmaschine transportiert und zwar in einer keimarmen oder keimfreien Atmosphäre, wie sie in der Kammer 37 vorherrscht.

Anstelle des Förderbandes 7 können die Flaschen 1 auch durch einen oder mehrere Transportsterne teilungsgerecht transportiert werden. Durch diese Blockbauweise können die Injektionsmaschine 5 und die Spülmaschine 6 unmittelbar nebeneinander mit geringstem Raumbedarf angeordnet werden. Auch ist es möglich, die Flaschen 1 direkt vom Auslaufstern 16a der Spülmaschine an den Einlaufstern der Füll- und Verschließmaschine zu übergeben. Auch bei dieser Bauweise lassen sich durch entsprechende Dimensionierung der Transpörtsterne die gewünschten Behandlungs- bzw. Wirkzeiten erzielen. Insbesondere bei geringeren Sterilitätsanforderungen ist es auch denkbar, die Mischdüsen 10 auf einem mit dem Einlaufstern 15a der Spülmaschine 6 rotierenden Drehtisch anzubringen, wobei die Einwirkzeit in den Bereich des Drehtisches 11a zu verlegen ist. In allen Fällen wird durch die direkte Beaufschlagung der Flaschen 1 durch das aus den Mischdüsen 10 austretende Desinfektionsmittel-Wasserdampf-Gemisch eine sparsame, zuverlässige und reproduzierbare Sterilisationsbehandlung ermöglicht.

## Patentansprüche

1. Verfahren zum Sterilisieren von verpackungsbehältern, wobei unter verwendung einer Düse ein erwärmtes Desinfektionsmittel-Wasserdampf-Gemisch erzeugt, den verpackungsbehältern zugeführt und nach Einwirkung auf die zu sterilisierende Oberfläche wieder entfernt wird, **dadurch gekennzeichnet, dass** einer Mischdüse gleichzeitig ein flüssiges Desinfektionsmittel und wasserdampf getrennt zugeführt wird, dass durch die Mischdüse ein Gemisch aus vernebeltem und/oder verdampftem Desinfektionsmittel und Wasserdampf gebildet wird und dass der die Mischdüse verlassende Gemisch-Strahl direkt auf und/oder in einen Verpackungsbehälter gerichtet wird.

2. Verfahren nach Anspruch 1, **dadurch gekennzeichnet, dass** das Mischungsverhältnis zwischen dem flüssigen Desinfektionsmittel und dem Wasserdampf ca. 2:1 beträgt.

3. Verfahren nach Anspruch 1 oder 2, **dadurch gekennzeichnet, dass** der der Mischdüse zugeführte Wasserdampf einen Druck von ca. 2 Bar und eine Temperatur von ca. 121 Grad Celsius aufweist.

4. Verfahren nach einem der Ansprüche 1 bis 3, **dadurch gekennzeichnet, dass** das der Mischdüse zugeführte Desinfektionsmittel Raumtemperatur aufweist.

5. Verfahren nach einem der Ansprüche 1 bis 4, **dadurch gekennzeichnet, dass** die Verpackungsbehälter vor der Beaufschlagung durch den aus der Mischdüse austretenden Gemisch-Strahl Raumtemperatur aufweisen.

6. verfahren nach einem der Ansprüche 1 bis 5, **dadurch gekennzeichnet, dass** das der Mischdüse zugeführte Desinfektionsmittel durch eine wässrige Lösung von H2O2, Peressigsäure und ggf. einem Tensid gebildet wird.

7. Verfahren nach einem der Ansprüche 1 bis 6, **dadurch gekennzeichnet, dass** die Sprühzeit der Mischdüse pro Verpackungsbehälter ein bis zwei Sekunden beträgt.

8. Verfahren nach einem der Ansprüche 1 bis 7, **dadurch gekennzeichnet, dass** die Einwirkzeit des auf dem Verpackungsbehälter kondensierten Desinfektionsmittel-Dampf-Nebels ca. fünf bis zehn Sekunden beträgt.

9. verfahren nach einem der Ansprüche 1 bis 8, **dadurch gekennzeichnet, dass** vor dem Entfernen des am Verpackungsbehälter niedergeschlagenen Kondensats eine Spülung der Verpackungsbehälter mit flüssigem Desinfektionsmittel erfolgt.

10. Verfahren nach einem der Ansprüche 1 bis 9, **dadurch gekennzeichnet, dass** die Flasche und die dieser zugeordnete Mischdüse während des Aufblasens des Desinfektionsmittel-Wasserdampf-Gemisches relativ zueinander stillstehen.

11. Verfahren nach Anspruch 1, **dadurch gekennzeichnet, dass** die Flasche und die zugeordnete Mischdüse während des Aufblasens des Desinfektionsmittel-Wasserdampf-Gemisches gemeinsam kontinuierlich translatorisch bewegt werden.

12. Verfahren nach einem der Ansprüche 1 bis 11, **dadurch gekennzeichnet, dass** der Gemischstrahl direkt durch die Mischdüse ins innere des Verpackungsbehälters eingeblasen wird, vorzugsweise durch dessen Mündungsöffnung.

13. Vorrichtung zur Durchführung des Verfahrens nach Anspruch 1, mit einem Förderer für die zu sterilisierenden Verpackungsbehälter und mindestens einer Düse, **dadurch gekennzeichnet, dass** mindestens eine Mischdüse (2) direkt auf die vom Förderer (11) transportierten Verpackungsbehälter (1) gerichtet ist, dass ein erster Kanal (18) der Mischdüse (2) über eine erste Leitung (22) und ein erstes Steuerventil (20) mit einem Wasserdampferzeuger (24) und ein zweiter Kanal (19) der Mischdüse (2) über eine zweite Leitung (23) und ein zweites Steuerventil (21) mit einem vorratsbehälter (26) für ein flüssiges Desinfektionsmittel (3) verbunden ist, und dass die Mischdüse (2) bei gleichzeitig öffnenden Steuerventilen (20, 21) ein Gemisch aus vernebeltem und/oder verdampftem Desinfektionsmittel und wasserdampf bildet.

14. Verfahren nach Anspruch 13, **dadurch gekennzeichnet, dass** die Mischdüse (2) als Zwei- Komponenten- Zerstäuberdüse ausgebildet ist.

15. Vorrichtung nach Anspruch 13 oder 14, **dadurch gekennzeichnet, dass** die Mischdüse (2) auf die Mündungsöffnung eines verpackungsbehälters (1) gerichtet ist.

16. Vorrichtung nach einem der Ansprüche 13 bis 15, **dadurch gekennzeichnet, dass** der Förderer (11) die Verpackungsbehälter (1) in aufrechter Position und horizontaler Richtung transportiert und die Mischdüse (2) senkrecht nach unten auf den Verpackungsbehälter (1) gerichtet ist.

17. Vorrichtung nach Anspruch 18, **dadurch gekennzeichnet, dass** der Förderer (11) kontinuierlich antreibbar ist und dass mehrere mit dem Förderer (11) mitlaufende mischdüsen (2) vorgesehen sind.

18. Vorrichtung nach Anspruch 19, **dadurch gekennzeichnet, dass** der Förderer (11) als Rotor ausgebildet ist, der am Umfang mehrere Halter (13) für die Verpackungsbehälter (1) trägt und dass auf dem Förderer (11) mehrere Mischdüsen (2) angeordnet sind, mindestens eine über jedem Halter (13).

19. Vorrichtung nach Anspruch 18, **dadurch gekennzeichnet, dass** die jeder Mischdüse (2) zugeordneten Steuerventile (20, 21) auf dem Förderer (11) angeordnet sind und unter Zwischenschaltung eines Drehverteilers (27) sowie über Leitungen (22, 23) mit dem Wasserdampferzeuger (24) und dem Vorratsbehälter (26) verbunden sind.

## Claims

1. Method for sterilization of packaging containers, wherein a nozzle is used to produce a heated disinfectant-steam mixture which is supplied to the packaging containers and removed after treating the surface to be sterilized, **characterized in that** liquid disinfectant and steam are separately fed to a mixing nozzle, **in that** a mixture of atomized and/or vaporized disinfectant and steam is formed and **in that** the mixture jet leaving the mixing nozzle is trained directly on to and/or into a packaging container.

2. Method according to Claim 1, **characterized in that** the mixing ratio between the liquid disinfectant and steam is approx 2:1.

3. Method according to Claim 1 or Claim 2, **characterized in that** the steam fed to the mixing nozzle is at a pressure of approx. 2 bar and a temperature of approx. 121° Celsius.

4. Method according to any one of Claims 1 to 3, **characterized in that** the disinfectant fed to the mixing nozzle is at room temperature.

5. Method according to any one of Claims 1 to 4, **characterized in that** prior to being treated with the mixture jet emerging from the mixing nozzle, the packaging containers are at room temperature.

6. Method according to any one of Claims 1 to 5, **characterized in that** the disinfectant fed to the mixing nozzle is formed by an aqueous solution of hydrogen peroxide, peracetic acid and optionally a tenside.

7. Method according to any one of Claims 1 to 6, **characterized in that** the spraying time of the mixing nozzle is one to two seconds per packaging container.

8. Method according to any one of Claims 1 to 7, **characterized in that** the treatment time of the disinfectant-steam mist condensed on the packaging container is approx. five to ten seconds.

9. Method according to any one of Claims 1 to 8, **characterized in that** the packaging containers are rinsed with liquid disinfectant prior to removal of the condensate deposited on the packaging container.

10. Method according to any one of Claims 1 to 9, **characterized in that** the bottle and the mixing nozzle assigned to it are stationary relatively to each other during the blowing of the disinfectant-steam mixture.

11. Method according to Claim 1, **characterized in that** the bottle and the mixing nozzle assigned to it are continuously traversed together during the blowing of the disinfectant-steam mixture.

12. Method according to any one of Claims 1 to 11, **characterized in that** the mixture jet is blown directly into the interior of the packaging container by the mixing nozzle, preferably through the mouth of the container.

13. Apparatus for carrying out the method according to Claim 1, with a conveyor for the packaging containers to be sterilized and at least one nozzle, **characterized in that** at least one mixing nozzle (2) is trained directly on the packaging containers (1) transported by the conveyor (11), **in that** a first passageway (18) of the mixing nozzle (2) is connected via a first line (22) and a first control valve (20) to a steam generator (24) and a second passageway (19) of the mixing nozzle (2) is connected via a second line (23) and a second control valve (21) to a feed tank (26) for liquid disinfectant (3), and **in that** the mixing nozzle (2) produces a mixture of atomized and/or vaporized disinfectant and steam when the control valves (20, 21) are opened simultaneously.

14. Method [sic] according to Claim 13, **characterized in that** the mixing nozzle (2) is configured as a two-component atomizing nozzle.

15. Apparatus according to Claim 13 or Claim 14, **characterized in that** the mixing nozzle (2) is trained on the mouth of a packaging container (1).

16. Apparatus according to any one of Claims 13 to 15, **characterized in that** the conveyor (11) transports the packaging containers (1) in the upright position and in a horizontal direction and the mixing nozzle (2) is directed vertically downwards towards the packaging container (1).

17. Apparatus according to Claim 18 [sic], **characterized in that** the conveyor (11) can be driven continuously and **in that** a plurality of mixing nozzles (2) travelling with the conveyor (11) are provided.

18. Apparatus according to Claim 19 [*sic*], **characterized in that** the conveyor (11) is configured as a rotor which carries a plurality of holders (13) for packaging containers (1) at its periphery and **in that** a plurality of mixing nozzles (2) are arranged on the conveyor (11), at least one above each holder (13).

19. Apparatus according to Claim 18, **characterized in that** the control valves (20, 21) assigned to each mixing nozzle (2) are arranged on the conveyor (11) and are connected, through a revolving distributor (27) and via lines (22, 23), to the steam generator (24) and feed tank (26).

## Revendications

1. Procédé de stérilisation de caisses d'emballage, au cours duquel, si l'on utilise une tuyère, un mélange désinfectant-vapeur d'eau est produit, est introduit dans les caisses d'emballage et est à nouveau enlevé après avoir agit sur la surface à stériliser,
**caractérisé en ce qu'**
un désinfectant liquide et de la vapeur d'eau sont simultanément et séparément introduits dans une tuyère mélangeuse, par laquelle un mélange de désinfectant atomisé et/ou vaporisé et de vapeur d'eau se forme de sorte que le mélange qui sort de la tuyère mélangeuse est directement dirigé sur et/ou dans une caisse d'emballage.

2. Procédé selon la revendication 1,
**caractérisé en ce que**
le rapport des ingrédients entre le désinfectant liquide et la vapeur d'eau est de 2:1.

3. Procédé selon la revendication 1 ou 2,
**caractérisé en ce que**
la vapeur d'eau introduite dans la tuyère mélangeuse présente une pression d'environ 2 bars et une température d'environ 121 degrés Celsius.

4. Procédé selon l'une des revendications 1 à 3,
**caractérisé en ce que**
le désinfectant introduit dans la tuyère mélangeuse est à température ambiante.

5. Procédé selon l'une des revendications 1 à 4,
**caractérisé en ce que**
les caisses d'emballage sont à température ambiante avant d'être alimentées par le mélange qui sort de la tuyère mélangeuse.

6. Procédé selon l'une des revendications 1 à 5,
**caractérisé en ce que**
le désinfectant introduit dans la tuyère mélangeuse est formé par une solution aqueuse composée de H202, d'acide peracétique et, le cas échéant, d'un agent tensioactif.

7. Procédé selon l'une des revendications 1 à 6,
**caractérisé en ce que**
la durée de pulvérisation de la tuyère mélangeuse est comprise entre une et deux secondes par caisse d'emballage.

8. Procédé selon l'une des revendications 1 à 7,
**caractérisé en ce que**
le temps d'imprégnation du brouillard de désinfectant et de vapeur condensé sur la caisse d'emballage est d'environ cinq à dix secondes.

9. Procédé selon l'une des revendications 1 à 8,
**caractérisé en ce que**
avant que le condensat déposé sur la caisse d'emballage ne soit enlevé, cette caisse d'emballage est nettoyée avec du désinfectant liquide.

10. Procédé selon l'une des revendications 1 à 9,
**caractérisé en ce que**
la bouteille et la tuyère mélangeuse associée sont arrêtées l'une par rapport à l'autre lors du soufflage du mélange désinfectant-vapeur d'eau.

11. Procédé selon la revendication 1,
**caractérisé en ce que**
la bouteille et la tuyère mélangeuse associée se déplacent toutes les deux par translation en continu lors du soufflage du mélange désinfectant-vapeur d'eau.

12. Procédé selon l'une des revendications 1 à 11,
**caractérisé en ce que**
le mélange est directement soufflé, via la tuyère mélangeuse, vers l'intérieur de la caisse d'emballage, de préférence via son orifice d'ouverture.

13. Dispositif de transport pour les caisses d'emballage à stériliser et équipé au moins d'une tuyère selon la revendication 1,
**caractérisé en ce qu'**
au moins une tuyère mélangeuse (2) est directement dirigée sur la caisse d'emballage (1) acheminée par le dispositif de transport (11), alors qu'un premier canal ( 18) de la tuyère mélangeuse (2) est raccordé, via une première conduite (22) et une première soupape de commande (20), à un producteur de vapeur d'eau (24) et un deuxième canal (19) de la tuyère mélangeuse (2) est raccordé, via une deuxième conduite (23) et une deuxième soupape de commande (21), à un réservoir (26) pour un désinfectant liquide (3), la tuyère mélangeuse (2) produisant, lorsque les soupapes de commande (20, 21) sont ouvertes simultanément, un mélange de désinfectant atomisé et/ ou vaporisé et de vapeur d'eau.

14. Procédé selon la revendication 13,
**caractérisé en ce que**
la tuyère mélangeuse (2) est équipée d'une tubulure de pulvérisation à deux composants.

15. Procédé selon la revendication 13 ou 14,
**caractérisé en ce que**
la tuyère mélangeuse (2) est dirigée sur l'ouverture d'orifice d'une caisse d'emballage (1).

16. Procédé selon l'une des revendications 13 à 15,
**caractérisé en ce que**
le dispositif de transport (11) achemine la caisse d'emballage (1) dans une position droite et dans une direction horizontale la tuyère mélangeuse (2) étant dirigée perpendiculairement vers le bas sur la caisse d'emballage (1).

17. Procédé selon la revendication 18,
**caractérisé en ce que**
le dispositif de transport (11) peut être actionné en continu et plusieurs tuyères mélangeuses (2) fonctionnant avec le dispositif de transport (11) sont prévues.

18. Procédé selon la revendication 19,
**caractérisé en ce que**
le dispositif de transport (11) est configuré comme un rotor qui comporte, sur sa périphérie, plusieurs supports (13) pour la caisse d'emballage (1) et, sur le dispositif de transport (11), sont placées plusieurs tuyères mélangeuses (2), au moins une sur chaque support (13).

19. Procédé selon la revendication 18,
**caractérisé en ce que**
les soupapes de commande (20, 21) associées à chaque tuyère mélangeuse (2) sont placées sur le dispositif de transport (11) et sont reliées, sous l'intercalage d'un distributeur rotatif (27), ainsi que via des conduites (22, 23), au producteur de vapeur d'eau (24) et au réservoir (26).
